# EUROPEAN PATENT SPECIFICATION

(11) **EP 1 013 725 B1**
(45) Date of publication and mention of the grant of the patent: **08.09.2004**
(21) Application number: 99125929.2
(22) Date of filing: 23.12.1999
(51) Int. Cl.: C09C 1/00, C09D 11/02

(54) **Pigment mixture**
Pigment-Mischung
Mélange de pigments

(30) Priority: 23.12.1998 EP 98124474
(43) Date of publication of application: 28.06.2000
(73) Proprietor: MERCK PATENT GmbH, 64293 Darmstadt (DE)
(72) Inventor: Pfaff, Gerhard, Dr., 64839 Münster (DE); Schoen, Sabine, Dr., 64287 Darmstadt (DE); Shimizu, Kaiman, Iwaki-shi, Fukushima-pref, 974-8251 (JP)

(56) References cited:
- EP-A- 0 659 843

## Description

The present invention relates to pigment mixtures containing at least two components, component A being Al₂O₃ flakes coated with one or more metals, metal oxides, and/or metal sulfides and component B being acicular or spherical colourants, and to their use in varnishes, paints, printing inks, plastics, powder coating materials and cosmetic formulations.

With platelet-shaped pigments, hiding power and gloss are often difficult to realize simultaneously to a satisfactory extent. For instance, SiO₂ flakes or mica platelets covered with one or more thin metal oxide layers feature interference colours and a high lustre but at the same time, owing to the transparent substrate, feature high transparency and hence a comparatively poor hiding power.

DE-A-42 40 511 discloses a pigment mixture which is composed of an interference pigment and a platelet-shaped colour pigment. The interference pigment comprises mica flakes or SiO₂ flakes coated with metal oxides and the colour pigment can be coloured, uncoated SiO₂ flakes. This pigment mixture is incorporated into coating materials, printing inks or plastics.

It is the object of the present invention to provide a pigment mixture which is notable for a comparatively high hiding power, which lends itself well to incorporation into the respective system in which it is used and for which at the same time the separation of pigment/colourant in the system is substantially ruled out.

Surprisingly, a pigment mixture has now been found which has none of the disadvantages indicated above. The pigment mixture of the invention consists of at least two components, component A being Al₂O₃ flakes coated with one or more metals, metal oxides and/or metal sulfides and component B being acicular or spherical colourants.

By admixing the colourant to the coated Al₂O₃ flakes it is possible to give the systems in which they are used a multiple flop, the colour effect is intensified, and new colour effects are achieved.

The invention thus provides a pigment mixture containing at least two components, component A being Al₂O₃ flakes coated with one or more metals, metal oxides and/or metal sulfides and component B being acicular or spherical colourants and that component A and component B are mixed in a ratio of 10:1 to 1:10.

The invention likewise provides the formulations, such as paints, varnishes, printing inks, plastics, powder coating materials and cosmetic formulations, which comprise the pigment mixture of the invention.

The coated Al₂O₃ flakes can be mixed with the colourant in the ratio from 1:10 to 10:1, in particular from 1:2 to 2:1.

The most important constituent of the inventive pigment mixture is the Al₂O₃ flake. Aluminum oxide in a flaky form is commercial available for example from Merck KGaA under the tradename Xirallic®.

α-Al₂O₃ in the form of hexagonal flakes having a particle diameter greater than 10 µm and an aspect ratio (particle diameter/thickness) of 5-10 is known from JP 111239/1982 (Laid Open No.).

The Japanese Patent Publication No. 72527/1991 discloses α- Al₂O₃ in the form of flakes having an average particle diameter of 0,5-3 µm.

The JP 39362/1992 (Laid Open No.) describes Al₂O₃ in the form of fine platy particles of a hexagonal crystal system with the plane perpendicular to the c axis grown into a plate.

Preferred Al₂O₃ flakes are flakes composed of aluminum oxide (as a major constituent) and of titanium dioxide (as a minor constituent) which are known from U.S. 5,702,519. These Al₂O₃ flakes are prepared from a uniform aqueous solution water-soluble aluminum salt and titanium salt by hydrolysis with an alkali carbonate aqueous solution in the presence of an aqueous solution containing an alkali metal salt like alkali metal sulfate and phosphoric acid or phosphate, drying by evaporation (dehydration by heating), and molten salt treatment.

The Al₂O₃ flakes are provided with one or more metal oxide layers. Examples of suitable metal oxides or metal oxide mixtures are titanium dioxide, zirconium oxide, zinc oxide, iron oxides (Fe₂O₃ and/or Fe₃O₄) and/or chromium oxide, especially TiO₂ and/or Fe₂O₃, as described in U.S. 5,702,519.

Coating of the Al₂O₃ flakes with a metal oxide may be accomplished by any known methods, such as hydrolysis of a metal salt by heating or alkali, which deposits hydrated metal oxide, followed by calcination.

Al₂O₃ flakes can also be coated with one or more layers of a metal or metal alloys selected e.g. from chromium, nickel, bismuth, copper, tin or hastalloy.

Al₂O₃ flakes coated with a metal sulfide are coated with sulfides e.g. of tungsten, molybdenum, cerium, lanthanum or rare earth elements.

The Al₂O₃ flakes can be coated by wet chemical coating, by CVD or PVD processes.

Colourants suitable as component B for the pigment mixture of the invention are all acicular and spherical colourants which are known to the skilled worker and have a particle size of from 0.001 to 20 µm, preferably from 0.01 to 3 µm. By acicular, fibre-like particles are meant those having a tength-to-diameter ratio of more than 5. Spherical colourants are here defined not only as ideal shaped spheres but also for particles which have more or less spherical shape of a non-uniform diameter. The pigment mixtures of the invention preferably comprise, as colourants, absorption materials and fillers.

The spherical colourants include, in particular, TiO₂, coloured SiO₂, CaSO₄, iron oxides, chromium oxides, carbon black, organic colour pigments, such as anthraquinone, quinacridone, diketopyrrolopyrrole, phthalocyanine, azo and isoindoline pigments. The acicular pigments preferably comprise BiOCl, coloured glass fibres, α-Fe₂O₃, α-FeOOH organic colour pigments, such as azo pigments, β-phthalocyanine CI Blue 15.3, Cromophtal Yellow 8GN (Ciba-Geigy), Irgalith Blue PD56 (Ciba-Geigy), azomethine copper complex CI Yellow 129, Irgazine Yellow 5GT (Ciba- Geigy).

The pigment mixture of the invention is simple and easy to handle. The pigment mixture can be incorporated into the system in which it is used simply by stirring it in. Laborious milling and dispersing of the pigments is not necessary.

The pigment mixture of the invention can be used for pigmenting coating materials, printing inks, plastics, agricultural films, the coating of seeds, food colourings, button pastes, medicament coatings or cosmetic formulations. The concentration of the pigment mixture in the system in which it is to be used for pigmenting is generally between 0.1 and 70 % by weight, preferably between 0.1 and 50 % by weight and, in particular, between 1.0 and 10 % by weight, based on the overall solids content of the system. It is generally dependent on the specific application.

Plastics comprising the pigment mixture of the invention in amounts of 0.01 to 50 % by weight, in particular from 0.1 to 7 % by weight, are frequently notable for a particular sparkle effect.

In the coating sector, especially in automotive finishing, the pigment mixture is employed - for 3-coat systems as well - in amounts of 0.1-10 % by weight, preferably from 1 to 3 % by weight. The proportion in which the coated Al₂O₃ flakes are mixed with component B depends on the desired effect. The Al₂O₃ flakes are preferably employed with component B in a proportion of 1:10 to 1:1, in particular of 1:3.

In the coating material, the pigment mixture of the invention has the advantage that certain colour flop effects can be achieved by a single-layer coating (one-coat system or basecoat in a two-coat system). This colour flop is pronounced even under diffuse light. In comparison with coating systems which comprise a mica-based interference pigment rather than the coated Al₂O₃ flakes, coating systems with the pigment mixture of the invention exhibit a more marked depth effect and a glitter effect.

The pigment mixture of the invention can also be employed in decorative and grooming cosmetology. The use concentration and the mixing proportion of Al₂O₃ flakes with component B, especially organic and inorganic colour pigments and dyes, of natural or synthetic origin, such as chromium oxide, ultramarine, or spherical SiO₂ or TiO₂ pigments, are dependent on the medium in which they are used and on the effect that is to be achieved. The Al₂O₃ flakes can be mixed with other pigments in any proportions, the preferred ratio being from 1:10 to 10:1. The use concentration ranges from 0.01 % by weight in a shampoo to 70 % by weight in a compact powder. In the case of a mixture of Al₂O₃ flakes with spherical fillers, such as SiO₂, the concentration in the formulation can be 0.01-70 % by weight. The cosmetic products, such as nail varnishes, lipsticks, compact powders, shampoos, loose powders and gels, are notable for particularly interesting lustre effects and/or colour effects. The glitter effect in nail varnish can be increased markedly relative to conventional nail varnishes with the aid of the pigment mixtures of the invention. Furthermore, the pigment mixture of the invention can be employed in bath products, in tooth pastes and for enhancing foods, for example as a mass colourant or as a coating.

In the pigmentation of binder systems, for example, paints and printing inks for intaglio, offset or screen printing, or as a precursor for printing inks, in the form for example of highly pigmented pastes, granules, pellets, etc., pigment mixtures, especially those consisting of coated Al₂O₃ flakes and spherical colourants, such as TiO₂, carbon black, chromium oxide, iron oxide and also organic absorption pigments, have been found particularly suitable. The pigment mixture is generally incorporated into the printing ink in amounts of 2-35 % by weight, preferably 5-25 % by weight and, in particular, 8-20 % by weight. Offset printing inks may comprise the pigment mixture in an amount of up to 40 % by weight or more. The precursors of printing inks, in the form for example of granules, pellets, briquettes, etc., contain up to 95 % by weight of the pigment mixture of the invention in addition to the binder and additives. The mixing ratio of component A to component B is preferably in the range from 1:10 to 10:1. The printing inks comprising the pigment mixture of the invention exhibit purer hues and their printability is improved owing to the good viscosity values.

The invention hence also provides formulations containing the pigment mixture of the invention.

The examples which follow are intended to illustrate the invention without, however, limiting it.

### Examples

### Example 1: - Printing ink

The pigment was incorporated into the solvent-containing binder by stirring at 600 rpm and the printing inks were subsequently knife-coated onto black-and-white cards.

| Ink No. 1: | |
|---|---|
| 88.0 g | Gebr. Schmidt 95 MB 011 TW |
| 10.0 g | Fe₂O₃-coated Al₂O₃ flakes of particle size 5 to 60 µm |
| 2.0 g | Gebr. Schmidt 95 MB 022-TW (Green) |

| Ink No. 2: Comparison | |
|---|---|
| 88.0 g | Gebr. Schmidt 95 MB 011 TW |
| 10.0 g | Fe₂O₃-coated mica of particle size 10 to 60 µm |
| 2.0 g | Gebr. Schmidt 95 MB 022-TW (Green). |

The colour card with ink No. 1 exhibits in visual terms a markedly better depth and glitter effect than the colour cards with the comparison ink No. 2.

### Example 2: - Automotive finish

| | |
|---|---|
| 2.0 g | Fe₂O₃-coated Al₂O₃ flakes of particle size 5-60 µm |
| 1.5 g | Heliogen Blue L 6930 |
| 0.2 g | Hostaperm Green 8G |
| 0.05 g | pigment-grade carbon black FW 200 |
| 66.6 g | basecoat (A4) MP system (FK = 19 %) |
| 29.65 g | diluent mixture |

### Example 3: - Plastic

Granules of the plastics polypropylene PP Stamylan PPH10 (from DSM) and polystyrene 143E (from BASF) are admixed in each case with
- a): 1 % of Fe₂O₃-coated Al₂O₃ flakes of particle size 5-60 µm
- b): a mixture of
1 % of Fe₂O₃-coated Al₂O₃ flakes of particle size 5-60 µm and
0.1 % of PV Fast Blue B2G01 (Pigment Blue 15.3 from Clariant)
- c): 1 % of Fe₂O₃-coated mica of particle size 10-60 µm
- d): a mixture of
1 % of Fe₂O₃-coated mica of particle size 10-60 µm and 0.1 % of PV Fast Blue B2G01 (Pigment Blue 15.3 from Clariant)

The pigmented granules are subsequently processed on an injection moulding machine to form small stepped plates. The plates differ in their color flops.

### Example 4: - Eye shadow

| Phase A | |
|---|---|
| 5.00 % | Silica |
| 25.00 % | TiO₂-coated Al₂O₃ flakes of particle size 5-60 µm (from Merck KGaA) |
| 5.00 % | CI Pigment Green 18 (CI77289) |
| 47.42 % | Talc |
| 7.18 % | Solanum Tuberosum (potato starch) |
| 2.40 % | Magnesium stearate |

| Phase B | |
|---|---|
| 6.96 % | Isopropyl stearate |
| 0.40 % | Cetyl palmitate |
| 0.40 % | Petrolatum |
| 0.08 % | Preservative |

The constituents of phase A are combined and formed into a premix. The melted phase B is then added dropwise with stirring to the powder mixture.

The powders are pressed at 40-50 bar.

### Example 5: - Lipstick

| Phase A | |
|---|---|
| 8.25 % | Cera alba |
| 4.95 % | Ceresin, Copernica Cerifera |
| 3.30 % | Lanolin oil |
| 5.28 % | Isopropyl myristate |
| 1.98 % | Mineral oil |
| 0.03 % | Tocopherol, ascorbyl palmitate, ascorbic acid, citric acid, PEG-8 |
| 0.06 % | Preservative |
| 0.50 % | Aroma |
| 1.00 % | Lithol rubine BK, C.I. Pigment Red 57:1 (CI 15850) |
| ad 100.00 % | Ricinus Communis (20 % in castor oil) |

| Phase B | |
|---|---|
| 2.00 % | Silica |
| 15.00 % | Iron oxide-coated Al₂O₃ flakes of particle size 5-60 *µm* (from Merck KGaA) |

The constituents of phase A are heated to 75° C and melted. The pigments of phase B are added and the entire batch is stirred thoroughly. The lipstick composition is then stirred for 15 minutes in the casting apparatus, which has been preheated to 65° C. The homogeneous melt is poured into the casting moulds, which have been preheated to 65° C. The moulds are then cooled and the cold mouldings removed.

## Claims

1. Pigment mixture containing at least two components, component A being Al₂O₃ flakes coated with one or more metals, metal oxides and/or metal sulfides and component B being acicular or spherical colourants and that component A and component B are mixed in a ratio of 10:1 to 1:10.

2. Pigment mixture according to Claim 1, **characterized in that** component A comprises TiO₂ and/or Fe₂O₃-coated Al₂O₃ flakes.

3. Pigment mixture according to Claim 1 or 2, **characterized in that** component B comprises coloured glass particles, carbon black, organic colour pigment and/or inorganic colour pigments.

4. Use of pigment mixture according to Claim 1 in varnishes, paints, automotive finihes, printing inks, plastics, powder coating materials, for colouring seed, in cosmetic formulations, and for enhancing foods.

5. Formulations containing a pigment mixture according to Claim 1.

## Patentansprüche

1. Pigmentgemisch enthaltend mindestens zwei Komponenten, wobei Komponente A mit einem oder mehreren Metallen, Metalloxiden und/oder Metallsulfiden beschichtete Al₂O₃-Plättchen und Komponente B nadel- oder kugelförmige Farbmittel sind und wobei Komponente A und Komponente B im Verhältnis von 10:1 bis 1:10 gemischt sind.

2. Pigmentgemisch nach Anspruch 1, **dadurch gekennzeichnet, dass** Komponente A mit TiO₂ und/oder Fe₂O₃ beschichtete Al₂O₃-Plättchen enthält.

3. Pigmentgemisch nach Anspruch 1 oder 2, **dadurch gekennzeichnet, dass** Komponente B farbige Glasteilchen, Ruß, organisches Farbpigment und/oder anorganische Farbpigmente enthält.

4. Verwendung des Pigmentgemisches nach Anspruch 1 in Lacken, Farben, Automobillacken, Druckfarben, Kunststoffen, Pulverlacken, zur Saatguteinfärbung, in kosmetischen Formulierungen und zur Veredelung von Lebensmitteln.

5. Formulierungen enthaltend ein Pigmentgemisch nach Anspruch 1.

## Revendications

1. Mélange de pigments contenant au moins deux composants, soit un composant A qui est constitué par des flocons d'Al₂O₃ revêtus d'un ou de plusieurs métaux, oxydes métalliques et/ou sulfures métalliques et un composant B qui est constitué par des colorants aciculaires ou sphériques, le composant A et le composant B étant mélangés selon un rapport de 10:1 à 1:10.

2. Mélange de pigments selon la revendication 1, **caractérisé en ce que** le composant A comprend des flocons d'Al₂O₃ revêtus de TiO₂ et/ou de Fe₂O₃.

3. Mélange de pigments selon la revendication 1 ou 2, **caractérisé en ce que** le composant B comprend des particules en verre colorées, du noir de charbon, un pigment de couleur organique et/ou des pigments de couleur inorganiques.

4. Utilisation d'un mélange de pigments selon la revendication 1 dans des vernis, des peintures, des finitions d'automobile, des encres d'impression, des matières plastiques, des matériaux de revêtement pulvérulents, pour colorer des germes, dans des formulations cosmétiques et pour ennoblix des aliments.

5. Formulations contenant un mélange de pigments selon la revendication 1.
